# EUROPÄISCHE PATENTSCHRIFT

(11) **EP 1 184 801 B1**
(45) Veröffentlichungstag und Bekanntmachung des Hinweises auf die Patenterteilung: **23.03.2005**
(21) Anmeldenummer: 01120358.5
(22) Anmeldetag: 24.08.2001
(51) Int. Cl.: G06F 19/00, G06F 1/00, H04L 9/00, H04L 29/06

(54) **Telemedizin-System**
Medical teleprocessing system
Système de tele-traitement médical

(30) Priorität: 29.08.2000 US 651578
(43) Veröffentlichungstag der Anmeldung: 06.03.2002
(73) Patentinhaber: SIEMENS AKTIENGESELLSCHAFT, 80333 München (DE)
(72) Erfinder: Abraham-Fuchs, Klaus, 91058 Erlangen (DE); Kleinschmidt, Peter, 91058 Erlangen (DE)

(56) Entgegenhaltungen:
- US-A- 4 757 534
- US-A- 5 222 137
- SCHNEIER B ET AL: "Remote auditing of software outputs using a trusted coprocessor" FUTURE GENERATIONS COMPUTER SYSTEMS, ELSEVIER SCIENCE PUBLISHERS. AMSTERDAM, NL, Bd. 13, Nr. 1, 1. Juli 1997 (1997-07-01), Seiten 9-18, XP004081705 ISSN: 0167-739X
- A. MENEZES, P. VAN OORSCHOT, S. VANSTONE: "Handbook of applied cryptography" 1997 , CRC PRESS , BOCA RATON, FL, USA XP002250236 * Seite 549 - Seite 550 *
- MAKRIS L ET AL: "Network access and data security design for telemedicine applications" COMPUTERS AND COMMUNICATIONS, 1997. PROCEEDINGS., SECOND IEEE SYMPOSIUM ON ALEXANDRIA, EGYPT 1-3 JULY 1997, LOS ALAMITOS, CA, USA,IEEE COMPUT. SOC, US, 1. Juli 1997 (1997-07-01), Seiten 523-527, XP010241407 ISBN: 0-8186-7852-6
- BLOBEL B ET AL: "CORBA security services for health information systems" INTERNATIONAL JOURNAL OF MEDICAL INFORMATICS, ELSEVIER SCIENTIFIC PUBLISHERS, SHANNON, IR, Bd. 52, Nr. 1-3, 1. Oktober 1998 (1998-10-01), Seiten 29-37, XP004153668 ISSN: 1386-5056

## Beschreibung

Die Erfindung bezieht sich auf ein Telemedizin-System zur zentralen Fernauswertung von medizinischen Patienteninformationen und -messdaten, basierend auf Einmalartikeln, in Weiteren auch disposables genannt, die mit Daten gekennzeichnet sind und/oder Messdaten erheben können.

Bei der telemedizinischen Behandlungsführung und Behandlungskontrolle über Netze - die Erfindung soll im Folgenden nur anhand ihrer Anwendung für medizinische Zwecke beschrieben werden, obgleich sie auch für nicht medizinische Zwecke einsetzbar ist - entstehen Probleme und neue Chancen. Problematisch dabei ist insbesondere die Datensicherheit. Es besteht die Gefahr das von Spähern im Netz die Behandlungsdaten mitgelesen werden und gegen die beteiligten Personen verwendet werden können. Noch schlimmer können die Folgen sein, wenn die Diagnose- und Behandlungsdaten von kriminellen Personen willentlich verändert werden. Aber auch das Risiko von versehentlicher Verwechslung von Arzt- und Patientenadressen muss bei einer solchen telemedizinischen Behandlungsführung reduziert werden. Darüber hinaus gibt eine telemedizinische Behandlungsführung durch die zentrale Zusammenführung der Behandlungsdaten die Möglichkeit fundiertere Aussagen über Wirkung von Methoden und Medikamenten zu gewinnen. Schließlich kann über eine telemedizinische Behandlungsführung auch schneller eine geographische Ausbreitung von Krankheiten erkannt werden.

Es ist naheliegend, die Daten bei der Übermittlung zu entschlüsseln, wobei sich auch hier wiederum das Problem stellt, dass solche Schlüssel sehr leicht geknackt werden können. Es gibt zwar zahlreiche Vorschläge, wie sichere Schlüssel (z. B. 128 bit) verwendet werden können und wie diese in aufwändiger Weise in Trust-Centern verwaltet werden, die auch die Echtheit der Absender garantieren. Darüber hinaus sind im Bankwesen auch Lösungen bekannt, bei denen die Authentifizierung durch die Zusendung von registrierten Transaktionsnummern realisiert wird. Sie müssen der Reihe nach abgearbeitet werden. Allen diesen Vorschlägen ist aber gemeinsam, dass sie sehr teuer sind und für die Beteiligten sehr umständlich sind.

In anderen Anwendungsgebieten existieren allerdings einfachere und effiziente Verschlüsselungsverfahren, die sich die Korrespondenz einer ID mit individuellen Codier-Schlüsseln, die den jeweiligen Datenelementen zugeordnet sind, zu Nutze machen.

Schneier et al. beschreiben in ihrem Artikel "Remote auditing of software outputs using a trusted coprocessor" (Future Generation Computer Systems, Bd. 13, Seiten 9 - 18, 1997) ein Verschlüsselungsverfahren, bei dem die verwendeten Softwareprogramme mit einem einen individuellen Codier-Schlüssel verschlüsselt sind und eine korrespondierende ID tragen. Mit Hilfe dieser ID kann effizient über eine Zentralstation für jedes Softwareprogramm der individuelle Codier-Schlüssel für die Entschlüsselung der Softwareprogramme erfragt werden. Eine Weiterverwendung des individuellen Codier-Schlüssels der jeweiligen Softwareprogramme für die Verschlüsselung und das Verschicken weiterer, aus der der Entschlüsselung der Softwareprogramme gewonnener Informationen ist jedoch in ihrer Veröffentlichung nicht vorgesehen.

Der Erfindung liegt daher die Aufgabe zugrunde, ein geeignetes Verschlüsselungsverfahren in einem Telemedizinsystem der eingangs genannten Art anzuwenden und die generierten Codier-Schlüssel effizient weiter zu verwenden.

Die Lösung dieser Aufgabe wird erfindungsgemäß durch die Merkmale des ersten Anspruchs gelöst.

Die Patientenstation kann sich dabei beim Patienten zuhause, beim Arzt, in einer Klinik, beim ambulanten Pflegedienst, beim Notfallarzt oder jeder anderen Stelle, an der sie der Patient nutzt, befinden. Der Hersteller des Disposables - gegebenenfalls kann es sich dabei auch um einen autorisierten Inverkehrbringer der Disposables handeln - meldet die individuellen Codier-Schlüssel jedes Disposables, das sich über seine Disposable-ID identifizieren lässt, über eine sichere (zertifizierte) Datenleitung an die Auswertestation, so dass diese dann mit Hilfe des individuellen Schlüssels die von der Patientenstation übermittelten Daten entschlüsseln und weiter verarbeiten kann.

Diese Weiterverarbeitung kann dabei insbesondere in der Weise erfolgen, dass nach der Auswertung der Disposable-Daten die Auswerte-Empfangsstation Diagnosen od. dgl. unter Verwendung des jeweils wechselnden individuellen Schlüssels des Disposables über die Datenleitung bzw. das Datennetzwerk an die Patientenstation zurückschickt.

Durch die Verschlüsselung der über ein Disposable gewonnenen Patientendaten über einen individuellen Schlüssel, der nur diesem einen Disposable zugeordnet ist, d. h. durch den Verzicht auf die Verwendung eines Schlüssels, mit dem alle Daten übersandt werden sollen, kann der Schlüssel sehr viel kürzer und einfacher gehalten werden, so dass die Anwendung sehr viel einfacher und billiger ist. Bei 128 bit-Schlüsseln ist ja in vielen Fällen der Schlüssel erheblich länger als die eigentliche zu verschlüsselnde Information. Über die Disposable-ID und die mit übertragene Kennung für die Patientenstation, also die Kennung für den jeweiligen behandelnden Arzt, die Klinik od. dgl. lässt sich die Information sicher an die Patientenstation zurückschicken, wo wiederum eine Entschlüsselung mit Hilfe des dort ja vorhandenen individuellen Schlüssels des Disposables möglich ist. Man hat also eine sehr sichere Datenübertragung ohne aufwendigen komplizierten Schlüssel und ohne die Notwendigkeit der Verwendung einer gesicherten Datenleitung.

Die Auswerte-Empfangsstation kann in Ausgestaltung der Erfindung über eine sichere Datenleitung mit dem Disposable-ID-Speicher des Disposable-Herstellers verbunden sein, was ja nur eine oder wenige sichere Datenleitungen von der oder den wenigen Auswerte-Empfangsstationen notwendig macht. Im Gegensatz dazu sind ja Tausende von Patientenstationen vorhanden, von denen sichere Datenleitungen zur Auswerte-Station praktisch nicht realisierbar sind.

In Weiterbildung der Erfindung kann vorgesehen sein, dass die Empfangsstation mit einer zentralen Patientendatenbank und/oder einem Expertensystem zur Auswertung der Daten anhand des abgespeicherten Fachwissens zur Erstellung einer individuellen Diagnose verbunden ist.

Das erfindungsgemäße Tele-Auswertesystem ermöglicht dabei auch eine Auslegung dahingehend, dass die Empfangsstation mit einem Abrechnungscomputer verbunden, der das Entgelt für die erbrachte Dienstleistung anhand der Disposable-ID und der Kennung der Patientenstation individuell abrechnet.

Darüber hinaus eröffnet das erfindungsgemäße Tele-Auswertesystem auch die Implementierung eines Sicherungssystems dergestalt, dass die Empfangsstation mit einer Speicher-Sperrstufe versehen ist, die Informationen von einem Einmal-Disposable, dessen ID bereits einmal bei einer Dateneingabe gemeldet war, aussperrt und/oder Meldung bei einer Überwachungsstelle erstattet. Auf diese Art und Weise lässt sich vermeiden, dass Disposables, die ja nur für eine einmalige Verwendung gedacht und ausgelegt sind, vom Patienten oder Arzt in unzulässiger Weise unter Gefährdung des Patienten und der Richtigkeit der ermittelten Daten nochmals eingesetzt werden.

In weiterer Ausgestaltung der Erfindung kann vorgesehen sein, dass bei der Abfrage des individuellen Schlüssels die Empfangsstation gleichzeitig Informationen über die Eichfaktoren von Disposable-Messgeräten und/oder Wirksamkeitsfaktoren und/oder die Herstelldaten von Medikamenten erhält. Damit ist beispielsweise ein komplizierter Abgleich von Messchips mit unterschiedlichen Eichfaktoren beim Disposable-Hersteller nicht erforderlich, sondern es genügt, dass die Eichkurven dort gespeichert und dem individuellen Disposable zugeordnet sind, so dass sie bei der Abfrage des individuellen Schlüssels mit übersandt und bei der Auswertung entsprechend mit berücksichtigt werden können.

Schließlich liegt es auch noch im Rahmen der Erfindung, dass die Empfangsstation ein neutrales, herstellerunabhängiges Health-Center ist, das die Patientendaten über Erfolg, Verträglichkeit, Nebenwirkungen od. dgl. einer Behandlung mit einem Disposable einerseits an die Patientenstation sowie anonymisiert an den Disposable-Hersteller weiterleitet. Dies ermöglicht die Gewinnung fundierter Aussagen über die Wirkung von Methoden und Medikamenten unter gleichzeitiger Gewährleistung der notwendigen Anonymität der Patienten.

Als Beispiele für Disposables, also für ein Einmal-Verbrauchsmaterial, das sich für ein erfindungsgemäßes Tele-Auswertesystem eignet, sind in erster Linie sogenannte diagnostische Bio-Chips zu nennen, beispielsweise Sensorchips, die durch Aufbringen eines Bluttropfens eine Vielzahl von Blutwerten erfassen und auswerten können.

Darüber hinaus können auch Medikamente und deren Individualverpackung erfindungsgemäß einsetzbare Disposables sein, wobei z. B. durch Codierung jeder Tablette individuell oder über einen individuellen Code für Verpackungsuntereinheiten od. dgl. eine Überprüfung stattfinden kann, ob es sich um die richtige Tablette handelt, ob die richtige Anzahl eingenommen worden ist usw. Auch Entnahmebestecke für Blut, Urin, Atemluft, also sogenannte Hygieneteile, können mit Hilfe des erfindungsgemäßen Tele-Auswertesystem besser auswertbare Disposables darstellen wie auf der anderen Seite auch Messelektroden, Diagnosemessstreifen, Hygieneauflagen (Papiere, Handschuhe usw.) für Mess- und Trainingsgeräte sowie Listen oder Dummies mit Transaktionsnummern und Schlüsselcodes.

Beispiele für die Anbringung und Auslesung von maschinellen Codier-Daten zum Aufbringen der Disposable-ID auf dem jeweiligen Disposable sind
- mechanische Muster, optisch sichtbar, alphanumerisch oder symbolisch vom Menschen lesbar und manuell, z. B. per Tastatur übertragbar,
- elektrische Kontaktmuster, die elektrisch abgreifbar sind,
- auslesbare Chips,
- Strichcode optisch einschließlich Farbe mit Stift, Scanner oder Bildverarbeitung decodierbar sowie Hochfrequenzresonatoren, die funktechnisch auslesbar sind,
- Magnetstreifen, die mit üblichen Kartenlesern auslesbar sind,
- biochemische, elektrische oder fluoreszenzoptisch auslesbare Marker usw.

Weitere Vorteile, Merkmale und Einzelheiten der Erfindung ergeben sich aus der nachfolgenden Beschreibung einiger Ausführungsbeispiele sowie anhand der Zeichnung. Dabei zeigen:
- Fig. 1: ein schematisches Blockschaltbild der teils verschlüsselten und unverschlüsselten Informationsübertragung zwischen den wesentlichen Stationen eines erfindungsgemäßen Tele-Auswertesystems, und
- Fig. 2: den schematischen Aufbau eines erweiterten erfindungsgemäßen telemedizinischen Disease Management-Systems.

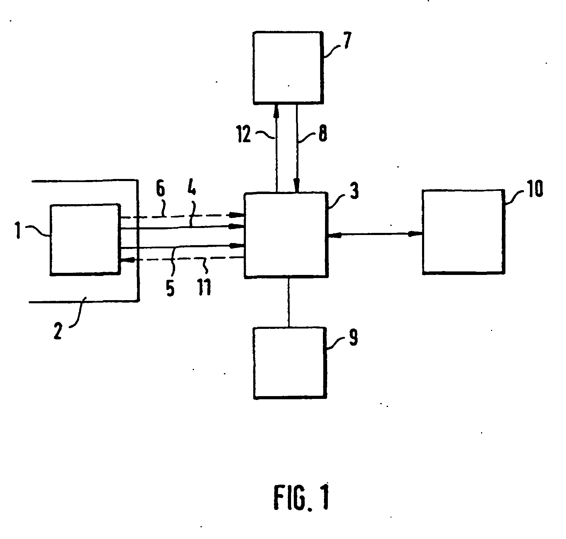

Von dem in Fig. 1 mit 1 bezeichneten Disposable, also beispielsweise einem diagnostischen Bio-Chip, wird die gewonnene Patienteninformation von der Patientenstation 2, also einer Sendestation beim Patienten zuhause, beim Arzt, in einer Klinik, beim ambulanten Pflegedienst, beim Notfallarzt od. dgl., über einen individuellen, nur dem jeweiligen Disposable 1 zugeordneten Schlüssel verschlüsselt, von der Patientenstation 2 an die Auswertestation 3 geschickt wird. Gleichzeitig mit dieser Versendung der verschlüsselten Disposable-Daten wird unverschlüsselt die Disposable-ID 5 zur Kennzeichnung des Disposables, von dem die Information gewonnen worden ist, mitübertragen und darüber hinaus erfolgt gegebenenfalls auch gleichzeitig eine Übertragung der Kennung 6 der Patientenstation 2. Die Auswertestation 3 ist beispielsweise über eine gesicherte Datenleitung mit dem Disposable-Hersteller 7 verbunden und erhält von diesem bei Angabe der Disposable-ID 5 den individuellen diesem Disposable zugeordneten Schlüssel als Information 8. Mit Hilfe dieses individuellen Schlüssels kann sie die Disposable-Daten 4 entschlüsseln und unter Verwendung gegebenenfalls einer zentralen Patientendatenbank 9 und eines Expertensystems 10 auswerten und eine entsprechende Diagnose erstellen. Diese kann dann als wiederum mit dem gleichen individuellen Schlüssel verschlüsselte Dateninformation 11 an die Patientenstation 2 zurückgeschickt werden, wo sie anhand des vorliegenden Schlüssels des Disposables 1 entschlüsselt und hier auch wieder dem jeweiligen Patienten zugeordnet werden kann. Die Daten über den Patienten müssen ja nicht zur Auswertestation mitgeliefert werden, es sei denn, dass man die Variante unter Verwendung einer zentralen Patientendatenbank 9 betreiben möchte. In diesem Fall könnte dann die Angabe des Patienten entweder unverschlüsselt erfolgen - wegen der Verschlüsselung der anderen wesentlichen Daten ist dies ja nicht sicherheitsrelevant- oder aber man könnte die Patientendaten mit dem gleichen individuellen Disposable-Schlüssel verschlüsseln.

Das schematisch in Fig. 1 skizzierte erfindungsgemäße Tele-Medizinsystem ermöglicht noch eine besonders sinnvolle und wesentliche Auswertung der vielen gesammelten Patientendaten dahingehend, dass sie als Daten 12 vom Auswertesystem 3 anonymisiert an den jeweiligen Disposable-Hersteller weitergeleitet werden - die Auswerte-Empfangsstation 3 steht selbstverständlich in Verbindung mit einer Vielzahl von Disposable-Herstellern - wobei jeder Disposable-Hersteller nur anonymisierte Patientendaten über Erfolg, Verträglichkeit, Nebenwirkungen od. dgl. einer Behandlung erhält, die mit Hilfe von Disposables aus seinem Haus gewonnen worden ist.

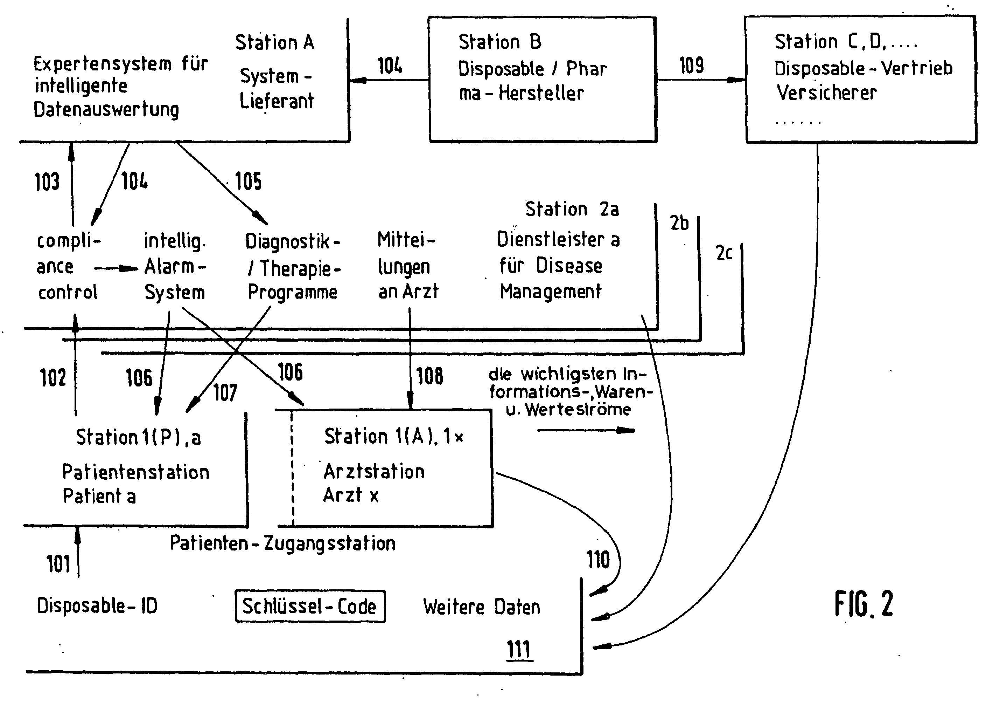

Die Fig. 2 zeigt schematisch ein telemedizinisches Disease Management-System, mit dem die bereits angesprochenen vielseitigen zusätzlichen Auswertemöglichkeiten der Patienteninformationen in umfänglicher Weise ausgenutzt werden können.

Die Bezugszeichen in Fig. 2 bedeuten dabei jeweils Folgendes:
- 101: bezeichnet die Datenübernahme vom Disposable,
- 102: die sichere Übertragung der mittels Einmal-Code verschlüsselten Daten,
- 103: die Übertragung der Diagnose-/Therapie-Ergebnisdaten zum zentralen Expertensystem,
- 104: das Versenden der Liste mit der Disposable-ID/Schlüssel/Disposable-Info (Medikamenten-Info),
- 105: rückzuübertragende Diagnostikwerte,
- 106: Patienteninformationen bei Unregelmäßigkeiten,
- 107: fortschrittsabhängige Information des Patienten und Versorgung mit neuen oder angepassten Vorsorge-/Therapieabschnitten,
- 108: regelmäßige Information zum behandelnden Arzt über Zustand und Fortschritt,
- 109: in Verkehr bringen der Disposables,
- 110: xEmpfehlung der Methode und Distribution der Mittel an den Patienten, und
- 111: Funktion des Disposables.

Unter der Funktion des Disposables 111 versteht man beispielsweise, dass es sich um ein Medikament, um ein Hygieneteil für Trainer oder Messgerät, Messelektroden, Teststreifen, Einmal-Sensoren, eine Entnahmevorrichtung od. dgl. handelt.

Die Erfindung ist nicht auf die dargestellten Ausführungsbeispiele beschränkt, so wäre es insbesondere auch noch möglich in das erfindungsgemäße Tele-Medizinsystem ein Abrechnungssystem zu implementieren oder aber auch Speichersperrstufen, die eine Mehrfach-Verwendung eines Disposables verhindern.

## Patentansprüche

1. Telemedizin-System zur zentralen Fernauswertung von medizinischer Patienteninformationen und -messdaten, basierend auf Einmalartikeln, die mit Daten gekennzeichnet sind und/oder Messdaten erheben können, **dadurch gekennzeichnet, dass** die Einmalartikel (1) einen individuellen Codier-Schlüssel tragen, um die Daten in einer Patientenstation (2) zu verschlüsseln, die per Datenleitung oder Datennetzwerk mit einer Speicher- und/oder Auswerte-Empfangsstation (3) verbunden ist, wobei gleichzeitig neben einer Kennung für die Patientenstation (2) eine auf dem Einmalartikel (1) angebrachte bzw. gespeicherte, in der Patientenstation automatisch mit erfassbare Einmalartikel-ID unverschlüsselt mit übertragen wird, und dass die Empfangsstation (3) mit dem Einmalartikel-Hersteller (7) datentechnisch verbunden ist, um über die Einmalartikel-ID den individuellen Codier-Schlüssel zum Entschlüsseln der Daten abzufragen und dass die Auswerte-Empfangsstation (3) Diagnosen od.dgl. unter Verwendung des jeweils wechselnden individuellen Codier-Schlüssels des Einmalartikels über die Datenleitung bzw. das Datennetzwerk an die Patientenstation (2) zurückschickt.

2. Telemedizin-System nach Anspruch 2, **dadurch gekennzeichnet, dass** die Auswerte-Empfangsstation (3) über eine sichere Datenleitung mit dem Einmalartikel-ID-Speicher des Einmalartikel-Herstellers (7) verbunden ist.

3. Telemedizin-System nach Anspruch 1 oder 2, **dadurch gekennzeichnet, dass** bei der Abfrage des individuellen Codier-Schlüssels die Empfangsstation (3) gleichzeitig Informationen über die Eichfaktoren von Einmalartikel-Messgeräten und/oder Wirksamkeitsfaktoren und/oder die Herstelldaten von Medikamenten erhält.

4. Telemedizin-System nach einem der Ansprüche 1 bis 3, **dadurch gekennzeichnet, dass** die Empfangsstation (3) mit einer zentralen Patientendatenbank (9) verbunden, insbesondere in diese integriert, ist.

5. Telemedizin-System nach einem der Ansprüche 1 bis 4, **dadurch gekennzeichnet, dass** die Empfangsstation (3) mit einem Expertensystem (10) verbunden ist.

6. Telemedizin-System nach einem der Ansprüche 1 bis 5, **dadurch gekennzeichnet, dass** die Empfangsstation (3) mit einem Abrechnungscomputer verbunden ist, der das Entgeld für die erbrachte Dienstleistung anhand der Einmalartikel-ID und der Kennung der Patientenstation (2) individuell abrechnet.

7. Telemedizin-System nach einem der Ansprüche 1 bis 6, **dadurch gekennzeichnet, dass** die Empfangsstation (3) mit einer Speicher-Sperrstufe versehen ist, die Informationen von einem Einmalartikel, dessen ID bereits einmal bei einer Dateneingabe gemeldet war, aussperrt und/oder Meldung bei einer Überwachungsstelle erstattet.

8. Telemedizin-System nach einem der Ansprüche 1 bis 7, **dadurch gekennzeichnet, dass** die Empfangsstation (3) ein neutrales herstellerunabhängiges Health-Center ist, das die Patientendaten über Erfolg, Verträglichkeit, Nebenwirkungen od. dgl. einer Behandlung mit einem Einmalartikel an die Patientenstation (2) sowie anonymisiert an den Einmalartikel-Hersteller (7) weiterleitet.

## Claims

1. Telemedicine system for centralised remote evaluation of medical patient information and measurement data, based on disposables (articles for one-time use) which are characterised with data and/or can gather measurement data, **characterised in that** the disposables (1) carry a unique encoding key for encrypting data in a patient station (2) which is connected via a data line or data network to a memory and/or evaluation receiving station (3), with in addition to an identifier for the patient station (2) a disposable ID which is also readable automatically in the patient station and attached to or, as the case may be, stored on the disposable (1) being transmitted unencrypted at the same time, and that the receiving station (3) is connected via a data link to the disposables manufacturer (7) in order to query, via the disposable ID, the unique encoding key for the purpose of decrypting the data and that the evaluation receiving station (3) sends back diagnoses or similar using the unique encoding key, which changes in each case, of the disposable via the data line or data network to the patient station (2).

2. Telemedicine system according to claim 1, **characterised in that** the evaluation receiving station (3) is connected to the disposable ID memory of the disposables manufacturer via a secure data line.

3. Telemedicine system according to claim 1 or 2, **characterised in that** when querying the unique encoding key the receiving station (3) simultaneously receives information relating to the calibration factors of disposable measurement equipment and/or effectiveness factors and/or the manufacturing data of medicines.

4. Telemedicine system according to one of the claims 1 to 3, **characterised in that** the receiving station (3) is connected to, in particular integrated with, a central patient database (9).

5. Telemedicine system according to one of the claims 1 to 4, **characterised in that** the receiving station (3) is connected to an expert system.

6. Telemedicine system according to one of the claims 1 to 5, **characterised in that** the receiving station (3) is connected to an accounting computer which individually calculates the fee for the service provided on the basis of the disposable ID and the identifier of the patient station (2).

7. Telemedicine system according to one of the claims 1 to 6, **characterised in that** the receiving station (3) is provided with a memory blocking stage which locks out information from a disposable whose ID has already been reported once before during a data input and/or sends a message to a monitoring centre.

8. Telemedicine system according to one of the claims 1 to 7, **characterised in that** the receiving station (3) is a neutral, manufacturer-independent health centre which forwards the patient data concerning success, compatibility, side effects or similar of a treatment by means of a disposable to the patient station (2) and, in anonymised form, to the disposables manufacturer (7).

## Revendications

1. Système de médecine à distance pour interpréter centralement à distance des informations médicales et des données de mesure liées à des patients et concernant des articles jetables qui sont **caractérisés par** des données et/ou qui peuvent fournir des données,
**caractérisé en ce que**
les articles jetables (1) portent une clé de codage individuelle pour coder les données dans un poste de patient (2) qui est relié à un poste de mémorisation et/ou de réception et d'interprétation (3) par des câbles de données ou par un réseau de données, en même temps outre l'indicatif pour le poste de patient (2) un identificateur d'article jetable qui est appliqué ou mémorisé sur l'article jetable (1) et qui peut être en même temps détecté automatiquement dans le poste de patient est alors simultanément transmis sans être codé
et le poste de réception (3) est relié par des moyens informatiques au fabricant (7) de l'article jetable afin de demander, grâce à l'identificateur d'article jetable, la clé de codage individuelle pour décoder les données
et le poste d'interprétation et de réception (3) renvoie au poste de patient (2) des diagnostics ou des éléments comparables en utilisant la clé de codage individuelle - qui change à chaque fois - de l'article jetable par l'intermédiaire du câble de données ou du réseau de données.

2. Système de médecine à distance selon la revendication 1, **caractérisé en ce que**
le poste d'interprétation et de réception (3) est relié à la mémoire des identificateurs d'article jetable chez le fabricant (7) de l'article jetable par l'intermédiaire d'un câble de données sûr.

3. Système de médecine à distance selon la revendication 1 ou 2,
**caractérisé en ce que**
lorsqu'on demande la clé de codage individuelle le poste de réception (3) obtient en même temps des informations concernant les facteurs d'étalonnage utilisés sur les appareils de mesure pour articles jetables et/ou les facteurs d'efficacité et/ou les données du fabricant au sujet des médicaments.

4. Système de médecine à distance selon l'une des revendications 1 à 3,
**caractérisé en ce que**
le poste de réception (3) est relié à une base de données " patients " centrale, en particulier y est intégré.

5. Système de médecine à distance selon l'une des revendications 1 à 4,
**caractérisé en ce que**
le poste de réception (3) est relié à un système expert (10).

6. Système de médecine à distance selon l'une des revendications 1 à 5,
**caractérisé en ce que**
le poste de réception (3) est relié à un ordinateur de règlement qui calcule individuellement le montant correspondant au service rendu à l'aide de l'identificateur de l'article jetable et de l'indicatif du poste de patient (2).

7. Système de médecine à distance selon l'une des revendications 1 à 6,
**caractérisé en ce que**
le poste de réception (3) comporte une étape qui bloque la mémoire pour bloquer les informations concernant un article jetable dont l'identificateur a déjà été indiqué par une entrée de données et/ou qui permet d'émettre un message auprès d'un poste de surveillance.

8. Système de médecine à distance selon l'une des revendications 1 à 7,
**caractérisé en ce que**
le poste de réception (3) est un centre de santé, neutre et indépendant du fabricant, qui transmet d'une part au poste de patient (2) et d'autre part sous forme anonyme au fabricant (7) de l'article jetable les données des patients où est indiqué si le traitement à l'aide d'un article jetable a réussi, s'il est bien supporté ou s'il a des effets secondaires etc.
